# EUROPEAN PATENT APPLICATION

(11) **EP 4 176 796 A1**
(43) Date of publication of application: **10.05.2023**
(21) Application number: 21206633.6
(22) Date of filing: 05.11.2021
(51) Int. Cl.: A61B 5/00, A61B 5/113, A61B 5/055, A61B 5/11, A61B 5/08

(54) **MULTI-SESSION BREATHING GUIDANCE**

(71) Applicant: Koninklijke Philips N.V., 5656 AG Eindhoven (NL)
(72) Inventor: ALBERTS, Eveline, Eindhoven (NL); HEUVELINK-MARCK, Annerieke, Eindhoven (NL)
(74) Representative: Philips Intellectual Property & Standards

(57) **Abstract**

A device (10), method and computer-program product are disclosed for providing breathing guidance to a subject during multiple diagnostic imaging and/or therapy sessions. The device comprises an input (11) for receiving sensor data and determining a respiratory parameter to monitor the respiration in each session. In a first session, a processor (14) determines when a breathing goal has been reached based on monitored respiratory parameter, and then determines and stores, using a data storage unit (13), a reference breathing pattern characterization of the monitored respiration. In a further session, the stored reference breathing pattern characterization is retrieved and feedback is provided to the subject via a feedback output (12) to influence the subject's breathing while monitoring the respiration to match and/or evolve toward the reference.

## Description

### FIELD OF THE INVENTION

The present invention relates to the field of breathing motion monitoring and/or control in medical procedures, such as during diagnostic imaging and/or therapeutical interventions. More specifically, the invention relates to a method, device and computer-program product for breathing guidance across multiple sessions, e.g. to create or promote a same or similar breathing pattern of the same patient during different imaging sessions (e.g. corresponding to different imaging modalities) and/or therapy sessions.

### BACKGROUND OF THE INVENTION

In many medical procedures, such as in diagnostic imaging, patient motion management can be advantageous. Patient motion is often a primary concern in diagnostic imaging and during treatment. Various sources of motion can be considered, some being more voluntary than others. Breathing motion is one type of motion that particularly needs to be taken into account when scanning the torso. For example, a common solution is to ask the examined patient to hold his/her breath while scanning. It is also well-known that coaching patients during their breath hold, e.g. by providing information about a remaining duration to reach a target or by providing a more sophisticated form of biofeedback, can improve the quality of the breath holds. Unfortunately, breath holds are not very comfortable or (easily) achievable for all patients. Therefore, a lot of development goes into imaging technology methods that can handle free breathing. The performance of such methods can vary, depending on the actual breathing pattern of the patient.

In diagnostic imaging, a regular breathing pattern can have a large effect on the quality of the acquired images. For example, knowledge of the breathing pattern, by measuring a relevant breathing-related parameter and/or by actively inducing a specific breathing pattern, may be exploited to collate different images or raw imaging data that correspond to, generally, a same breathing phase, and/or to annotate acquired data or images to indicate the corresponding breathing phase.

Likewise, the breathing pattern can strongly affect the quality of a medical treatment procedure and, hence, also its therapeutic outcome. For example, it is important in radiotherapy that a specific therapeutic radiation dose is delivered accurately to its intended target volume without excessive exposure of nearby tissues, e.g. of organs at risk, even though the target volume can often be subject to movement due to breathing.

The specific breathing parameter that influences the quality of the imaging and/or therapeutic procedure can also vary. A stable and/or predictable breathing amplitude may be important, as well as (additionally or alternatively) the breathing frequency and/or it's specific alignment in time with the procedure being performed, e.g. such that images can be acquired or a therapy can be delivered at the precise moment that the lungs are maximally emptied or filled (for example; i.e. without limitation thereto).

Motion management techniques can, particularly, include methods of breathing monitoring and guidance. It is known in the art that a breathing coach system may be advantageous in obtaining, i.e. inducing and/or promoting, a specific breathing pattern that is better suited for the particular procedure being performed, e.g. a regular breathing pattern while imaging or treating a patient. For example, visual, auditive and/or other (bio)feedback methods may be used to guide the patient toward a (more) regular breathing pattern.

It is well-known that a breathing-related physiological parameter can be monitored, and instructions such as a request to hold breath can be given to the patient based on the monitored parameter. This can, obviously, be performed manually, or can be automated by a system, such as the system disclosed in US 2013/0211236 A1.

Solutions that coach patients to breathe in a manner optimal for a particular application (e.g. a diagnostic imaging modality, such as magnetic resonance imaging or computed tomography, or a treatment, such as a radiotherapeutical method) are known in the art. Such solutions may include biofeedback and may be more or less adaptive to a specific patient. Specific goals of breathing coaching for a particular application could be breath holds at a particular amplitude, breathing with an extended end exhalation, or shallow consistent breathing. An example of such approach can be found in WO 2020/069948 A1, which discloses a breathing adaptation system for influencing a breathing parameter of a user's breathing pattern in line with a predetermined goal. A sensor measures a current value of the breathing parameter, and a feedback unit provides feedback about a preferred value of the influenced breathing parameter, in which the preferred value is determined by a control unit in a user-specific manner.

Likewise, in the field of radiotherapy, it is realized that reproducing exactly the same breath hold level (or best approximation thereof) by a patient across multiple days of treatment is desirable for optimal treatment outcomes, but can be very challenging to achieve. US 2020/038690 A1 discloses a method of customized breathing maneuver guidance during radiotherapy treatment by using an augmented reality system fitted to a treatment couch. A position measurement module measures a distance from a fixed position to a patient anatomic region during a breathing cycle, and the patient can monitor and control a state of their breathing cycle in real time from breath state information displayed on an instruction screen. A patient-customized breath hold amplitude is determined by measuring the distance between a baseline exhale position and a maximum inhale position, using the measurement module. The breath hold amplitude data is furthermore stored for subsequent breath hold guidance, such that it can be replicated in subsequent radiotherapy sessions for the same patient. However, even though the approach can be useful in reproducing a same breathing pattern across several radiotherapy sessions, it may be a disadvantage that this prior art approach is only suitable for implementing a single breathing paradigm, which aims to achieve maximally deep inhalation breath holds, whereas in practice the ideal breathing protocol can differ from case to case. Another disadvantage may be that an augmented reality system (measurement and feedback system) is used that can be attached via a universal clamping mechanism onto a wide variety of patient couches to allow the same system to be used in various sessions. However, this may also imply that valuable time could be lost in frequently removing and reinstalling the system, since the same system may need to be used for each session in possibly different treatment rooms or under different conditions.

### SUMMARY OF THE INVENTION

It is an object of embodiments of the present invention to provide in good and/or efficient (e.g. automated) breathing guidance in medical imaging and/or other medical procedures, e.g. to create or stimulate a same or similar breathing pattern in the same patient (or, generally, subject) in different sessions, e.g. different imaging and/or therapy sessions. For the purposes of the present disclosure, breathing guidance may also be referred to as breathing coaching, and vice versa, e.g. these terms may essentially refer to a same or similar process of influencing the respiration of a person, e.g. a patient, by providing relevant information (e.g. feedback information) to that person in accordance with an objective for the breathing behavior.

It is an advantage of embodiments of the present invention that breathing assistance (e.g. coaching) can be provided to the same subject during different imaging sessions, e.g. corresponding to different imaging modalities. Thus, a reproducible breathing pattern can be created across imaging modalities, which can be advantageous. For example, having the same breathing pattern during different imaging sessions, e.g. a CT scan and an MRI scan, and particularly when (each of) these scans are four-dimensional ("4D", i.e. temporospatial), can aid in mapping (e.g. co-registering) the image information between both modalities.

For example, in magnetic resonance imaging (or computed tomography), respiratory triggering or gating may be used, as known in the art, to obtain a more predictable scan time and enhanced image quality. These advantages can be further enhanced by active breathing management, e.g. in the form of breath guidance in accordance with embodiments of the present invention. It will be understood that applying the same breathing pattern guidance paradigm (for the same patient) in different imaging examinations, e.g. a (e.g. 4D) MRI and a (e.g. 4D) CT examination, results in the advantages of controlled, regular breathing as such for each imaging session independently, but also from advantages related to reproducible breathing behavior between these modalities, e.g. smoothening the image registration process, thus positively affecting the diagnostic process or treatment planning process relying on the collected image sequences. For example, the image registration process can advantageously be generally reduced in computational complexity, e.g. such that less iterations are needed to reach a solution of the registration problem (without limitation to iterative registration methods), and/or the risk of getting stuck in a globally sub-optimal yet local optimum of the registration optimization may be reduced.

It is an advantage of embodiments of the present invention that breathing assistance (e.g. coaching) can be provided to the same subject during one or more imaging sessions as well as during one or more treatment sessions. This can be advantageous in improving the accuracy of the treatment delivery, e.g. radiotherapy, by allowing a better real-time prediction or detection of the position of a target volume in the subject's body, since it's movement during therapy will correspond better to the image information acquired beforehand. Thus, the effectiveness and safety of the therapeutical produce, e.g. radiotherapy, may be improved.

It is an advantage of embodiments of the present invention that a good image quality can be achieved and/or a good prediction can be made of the scan and/or treatment time in one (or each) of the aforementioned sessions.

As a specific example, it will be understood that the advantage of embodiments of the present invention for cross-modality information mapping (including image registration) can be directly applied or transposed to a situation where a treatment is combined with live imaging, e.g. a radiotherapy system (e.g. using a linear accelerator for therapeutical radiation dose delivery) with portal imaging, CT imaging or MRI imaging system. Since the live imaging in such systems, as known in the art, can be used to guide (e.g. trigger or gate) the treatment delivery, a good registration of the live imaging feed to prerecorded imaging data can be highly advantageous to accurately predict or determine the optimal timing of the treatment delivery (or to control the treatment in another suitable manner). Thus, the exposure of healthy tissue to damaging radiation can be reduced.

Nonetheless, even if the radiotherapy system does not include a complex system for live imaging, it will be appreciated that it can still be advantageous to apply a same breathing coach paradigm across pre-treatment imaging sessions (e.g. for therapy planning) and one or more treatment sessions. Positioning the patient in a reproducible way for imaging and treatment is generally a key element in the radiotherapy workflow, such that, even when the radiotherapy is not actively guided (e.g. gated) by live imaging, position information about a target volume (and associated risk margins) will correspond better to the actual position during treatment when such information is based on images that are acquired under the same breathing conditions as during treatment.

When the same breathing behavior is reproduced during one or more imaging sessions as well as during the treatment (e.g. in one or more treatment sessions), this can influence the treatment itself in a positive way, and can lead to higher quality of care. In particular, reproducible breathing behavior will likely improve the accuracy of the delivered treatment plan. When the breathing guidance system coaches the patient to breathe in a consistent, shallow manner, this can also have further benefits due to a smaller treatment region (e.g. a reduced internal and/or planning target volume). In cases where a gated treatment approach is applied (e.g. on end-inhalation), coaching towards an inhalation level that is reproducible will likely also improve the quality and duration of the treatment. Furthermore, coaching the patient toward a prolonged end-inhalation state ("mini" breath hold), scan efficiency can also be improved.

This particularly illustrates that it is an advantage to provide a method and/or means that allow a specific goal to be set for the breathing paradigm as a function of the desired outcome or intended treatment protocol, and that is not necessarily optimal for the session in which it is (e.g. initially) applied, but rather optimized for a global multisession trajectory, e.g. taking a future therapy session into account in which the same breathing protocol will be repeated.

It is an advantage of embodiments of the present invention that the breathing entrainment protocol can be determined (e.g. varied) as function of the requirements. For example, the breathing pattern may be optimized for a specific imaging protocol to obtain good imaging results, or may be optimized for a specific therapy, e.g. a radiotherapy session. It is an advantage that the breathing protocol can be selected for optimal (or at least sufficient) results in a therapy session, while it can be applied to multiple sessions, e.g. one or more imaging sessions prior to the therapy session. This allows the subject (patient) to become familiar with, and eventually proficient in, the entrained breathing pattern before the actual therapy session. It is an advantage that the patient does not need to be trained in the breathing procedure in (a) separate, e.g. dedicated, session(s), i.e. that such pre-treatment training can be efficiently integrated with pre-treatment imaging sessions. This, however, does not preclude the use of additional (dedicated) training sessions in accordance with embodiments of the present invention, where any additional breathing training that is integrated in imaging sessions would clearly still carry an advantage in terms of increased overall efficiency.

As an example, a consistent, shallow breathing pattern may be advantageous for radiotherapy sessions where a small treatment region is to be treated. Applying this breathing pattern during (a) prior imaging session(s) may also reduce blurring of the image, such that the small region can be more precisely defined. In another example, a treatment plan may define a radiotherapy session using a gated approach on (for example) end-of-inhalation. Thus, prior training of the patient (during one or more earlier imaging sessions) to achieve a same reproducible inhalation level without large deviations of the end-inhalation air volume and/or to avoid sudden inhalation or exhalation bursts may greatly improve the therapy quality, and may also reduce the duration of the treatment (or may reduce some random variation of the session time).

For example, a target breathing pattern of the breath training may also coach the patient toward a short ("mini") breath hold (e.g. which can also be characterized as a prolonged end-exhalation and/or prolonged end-inhalation), to improve an imaging scan efficiency and/or to widen a temporal window in the (or each) breathing cycle during which (e.g. radio-)therapy can be accurately delivered/performed.

Whereas historically the focus of medical imaging and medical procedures in general has been, quite understandably, on patient health and safety, diagnostic accuracy, therapeutic efficacy and the like, a trend toward increased attention for the experience and comfort of the patient exists. This can contribute to an improved patient cooperation and to an efficient workflow. By creating an atmosphere that brings the patient from a clinical environment, with potentially fear and/or other negative feelings associated therewith, to a relaxing setting, the patient comfort and cooperation can be strongly enhanced, which can also improve the workflow, e.g. potentially increasing the throughput in terms of number of procedures that can be performed in a given time. Thus, attention to patient experience does not only have benefits in terms of contributing to a positive reputation as a medical provider, but also has very practical and tangible advantages.

Methods to improve the patient experience may particularly include (automated) attentive patient guidance to guide the patient through breath holds, e.g. in accordance with embodiments of the present invention. This breathing guidance may also be easily integrated with informative messages, such as to announce table movements (i.e. of the couch supporting the patient) and/or to indicate a scan duration or progress of a diagnostic (e.g. CT or MRI) imaging procedure being performed. This combination is particularly useful since, in addition to potential clinical advantages of achieving a better breathing pattern, a breathing guidance system can also be useful in distracting the patient from the procedure being performed by drawing the attention to his/her breathing, thus alleviating potential anxiety or nervosity. Likewise, actively engaging the patient in the imaging and/or therapy procedure may also improve the subjective experience by offering a sense of control and/or involvement to the patient.

It is an advantage of embodiments of the present invention that a high-quality radiotherapy treatment (e.g. in image-guided precision therapy) can be achieved by promoting reproducible breathing behavior across therapy sessions and/or by ensuring that images acquired for treatment planning are representative of the same breathing phase as will be used for treatment or of the same dynamic breathing pattern as will be encountered during treatment.

A method, device and computer-program product in accordance with embodiments of the present invention achieves the above objective.

In a first aspect, the present invention relates to a method for providing breathing guidance to a subject during multiple diagnostic imaging and/or therapy sessions of a multisession series for said subject. The method comprises, during each of said sessions, monitoring the respiration of the subject by repeatedly measuring at least one respiratory parameter. In a first session of the multisession series, it is determined when a predetermined or selected breathing goal has been reached based on the monitored at least one respiratory parameter. Furthermore, in this first session, a reference breathing pattern characterization of the monitored at least one respiratory parameter is determined (and stored) once it is determined that the breathing goal has been reached.

In at least one subsequent session of the multisession series, the stored reference breathing pattern characterization is retrieved, and feedback is provided to the subject to influence the subject's breathing while monitoring the respiration in order to match or approximate the reference breathing pattern characterization.

A method in accordance with embodiments of the present invention may comprise, in said first session, providing feedback to the subject before it is determined that said breathing goal is reached in order to influence the subject's breathing while monitoring the respiration to meet or evolve toward said breathing goal.

In a method in accordance with embodiments of the present invention, the step of providing feedback to the subject to influence the subject's breathing to match or approximate the reference breathing pattern characterization may also be performed in the first session after the reference breathing pattern characterization has been determined.

A method in accordance with embodiments of the present invention may also comprise, in said first session, automatically or by interaction with an operator, selecting the breathing goal from a plurality of different candidate breathing goals, in which the breathing goal defines at least one target criterium for said at least one respiratory parameter.

A method in accordance with embodiments of the present invention may comprise generating a trigger signal to indicate that the breathing goal has been reached to an imaging or therapeutical system, such as to commence with a scanning or therapy operation upon receipt of said trigger signal.

A method in accordance with embodiments of the present invention may comprise, in the at least one subsequent session, determining when the earlier stored reference breathing pattern is matched or sufficiently approximated by the subject's breathing and then generating a further trigger signal for an imaging or therapeutical system so as to commence with a scanning or therapy operation upon receipt of said further trigger signal.

A method in accordance with embodiments of the present invention may comprise generating a pause signal to interrupt the scanning or therapy operation when the subject's breathing deviates from the reference breathing pattern.

In a second aspect, the present invention relates to a computer-program product for performing, when executed on a computer, a method in accordance with the first aspect of the present invention.

In a third aspect, the present invention relates to a device for providing breathing guidance to a subject during multiple diagnostic imaging and/or therapy sessions of a multisession series for said subject. The device comprises an input for receiving sensor data and determining at least one respiratory parameter therefrom to continually monitor the respiration of the subject during each of said sessions, a feedback output for providing sensory feedback to the subject in each of said sessions, a data storage unit and a processor. The processor is adapted to, in a first session of said multisession series, determine when a predetermined or selected breathing goal has been reached based on the at least one respiratory parameter monitored via the input, and determine and store, using the data storage unit, a reference breathing pattern characterization of the monitored at least one respiratory parameter once it is determined that the breathing goal has been reached. The processor is also adapted to, in at least one subsequent session of the multisession series, retrieve the stored reference breathing pattern characterization from the data storage unit and provide feedback to the subject via the feedback output to influence the subject's breathing while monitoring the respiration via the input in order to match and/or evolve toward the reference breathing pattern characterization.

In a device in accordance with embodiments of the present invention, the processor may be adapted to, in said first session, before it is determined that said breathing goal is reached, provide feedback to the subject via the feedback output based on the at least one respiratory parameter monitored via the input in order to influence the subject's breathing to meet and/or evolve toward said breathing goal.

In a device in accordance with embodiments of the present invention, the processor may be adapted to, in said first session, after it is determined that said breathing goal is reached, provide feedback to the subject via the feedback output based on the at least one respiratory parameter monitored via the input in order to influence the subject's breathing to match and/or evolve toward the reference breathing pattern characterization.

A device in accordance with embodiments of the present invention may comprise a goal selector to, in said first session, automatically or by interaction with an operator select said breathing goal from a plurality of different candidate breathing goals, wherein said breathing goal defines at least one target criterium for said at least one respiratory parameter for use in determining when said breathing goal has been reached.

A device in accordance with embodiments of the present invention may comprise a trigger signal output, in which the processor may be adapted to generate a trigger signal for indicating that said breathing goal has been reached and/or said reference breathing pattern characterization has been matched by the subject's breathing to an imaging or therapeutical system via said trigger signal output, such as to commence with a scanning or therapy operation upon receipt of said trigger signal.

In a device in accordance with embodiments of the present invention, the processor may be adapted to generate a pause signal via the trigger signal output (or a further signal output) to interrupt the scanning or therapy operation when the subject's breathing deviates from the reference breathing pattern characterization.

A device in accordance with embodiments of the present invention may comprise multiple separate units, each unit comprising an instance of said input, said feedback output and said processor. The units may be operably connected to the (e.g. same) data storage unit, such that different units have access to the reference breathing pattern characterization and can respectively operate in different rooms and/or in combination with different imaging and/or therapy systems for provide breathing guidance in different sessions of the multisession series. One or more of the units may also be used in (i.e. may be adapted for use in) preparatory training of the breathing behavior, such as to practice the intended breathing behavior in a waiting room or even at home to prepare the patient for the imaging and/or therapy procedure at a later time.

In a device in accordance with embodiments of the present invention, at least two of said inputs may comprise a converter, e.g. different converters, adapted to receive sensor input from respectively different sensors and for determining the same at least one respiratory parameter therefrom, such that different units can receive input from different sensor types and derive the same at least one respiratory parameter therefrom.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a method in accordance with embodiments of the present invention.
Fig. 2 shows a device in accordance with embodiments of the present invention.
Fig. 3 schematically shows a first illustrative architecture of embodiments of the present invention.
Fig. 4 schematically shows a second illustrative architecture of embodiments of the present invention.
Fig. 5 outlines a typical radiotherapy workflow, to illustrate aspects of the present invention.
Fig. 6 shows a delineated target volume on a diagnostic image for use in a radiotherapy procedure, to illustrate aspects of the present invention.
Fig. 7 shows delineated organs at risk on a diagnostic image for use in a radiotherapy procedure, to illustrate aspects of the present invention.
Fig. 8 shows a composite mosaic of coregistered magnetic resonance and computed tomography images, to illustrate aspects of the present invention.
Fig. 9 schematically illustrates a breathing cycle and different time bins, indicated as "phases," to illustrate aspects of the present invention.
Fig. 10 schematically illustrates the creation of an averaged image from different respiratory phases, to illustrate aspects of the present invention.
Fig. 11 schematically shows various volume definitions commonly used in radiotherapy, to illustrate aspects of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a method for breath guidance for a subject's breathing across multiple sessions of a multisession series for a same subject (e.g. typically a patient), e.g. to create or promote a same or similar breathing pattern of the same patient during different imaging sessions and/or therapy sessions.

Particularly, the method may be a computer-implemented method, i.e. an automated or semi-automated method that can be performed by dedicated processing hardware (e.g. using an application specific integrated circuit or circuits) and/or configured/programmed processing hardware, such as a computer or other general purpose processor that is programmed for the specific task of performing the method and/or a configurable hardware platform (e.g. a field programmable gate array) configured for the specific task of performing the method. Combinations of application specific hardware (e.g. ASIC) and/or configured hardware (e.g. FPGA) and/or one or more programmed processing devices (e.g. using a CPU, GPU or other suitable processor, possibly in combination with supporting hardware, e.g. as commonly found in computers) are also possible.

'Semi-automated' may refer to a computer-implemented method that relies on (e.g. limited) input from and/or interaction with an operator, e.g. to select or enter relevant parameters, but otherwise may act in an autonomous or supervised autonomous mode. Input from an operator can refer to interactive input, e.g. by means of a (e.g. graphical) user interface, but can also refer to input that is retrieved from a storage device or via an external controller, e.g. externally (possibly remotely) entered or even prerecorded at an earlier planning stage.

It will be understood that the multisession series may be unified by a same treatment goal, for example to treat a specific disease, such as a specific cancer (e.g. a specific tumor or malignancy) and/or to treat conditions affecting a specific body part, such as a clinical case presenting a combination of cancerous tissues in a same body region, and/or a disease or diseases that are occurring in the patient at the same time and can be treated in a unified treatment plan (e.g. a metastasized cancer). However, it is to be noted that embodiments are not necessarily limited to radiotherapy treatments nor to oncological treatments. For example, other illustrative treatments may include (e.g. MR-guided) high-intensity focused ultrasound (HIFU) tissue ablation procedures, for example to treat uterine fibroids and/or other applications in the female pelvic region, and/or other (e.g. image-guided) ablation procedures. Particularly, the multisession series may comprise not only one or more treatments in line with the intended treatment goal, but may also comprise one or more diagnostic imaging sessions, e.g. to obtain detailed information for the purpose of planning the treatment.

For example, the multisession series may comprise an imaging session, e.g. a magnetic resonance imaging, computed tomography, positron emission tomography, single photon emission computed tomography, echography (or ultrasound imaging), and/or other imaging modality session, in combination with a treatment session. The multisession series may comprise more than one imaging session, e.g. sessions that are separated in time and/or space from each other, e.g. performed in different rooms and/or using different systems and/or on different days, or at least separated by the patient leaving the imaging environment and returning to the same or another imaging environment on another occasion for the next session. Thus, one advantage of embodiments of the present invention is that a reproducible breathing pattern can be achieved in multiple (e.g. 4D) imaging sessions, e.g. using multiple imaging technologies, such as CT, MRI, PET/SPECT, ultrasound imaging and/or in imaging combined with therapy, e.g. MR-Linac.

Likewise, the multisession may comprise more than one treatment session, e.g. likewise clearly separated in time and/or space, e.g. on different days and/or separated by (at least) the patient leaving his/her position on a treatment couch and returning later to the same or a different treatment room. As already mentioned, the (or one of the) treatment session(s) may refer to a radiotherapy session, e.g. using a conventional high-energy photon irradiation (e.g. using a linear accelerator). Radiotherapy may also refer to a proton therapy or other type of hadron therapy, an electron irradiation, or, generally, other type of radiotherapy. The treatment may also, as already mentioned, refer to different types of treatments, e.g. (without limitation) a stone ablation procedure, a biopsy and/or a precision treatment, e.g. especially when the treatment is strongly automated (without limitation, e.g. some manual intervention and/or human supervision may typically be still implied in therapeutical treatments) and/or strongly dependent on imaging technology (e.g. robotic surgery and/or image-guided precision therapy). A link between imaging and therapy may be direct, e.g. as in live guidance by imaging during the treatment, or indirect, e.g. as relying on pre-acquired images to accurately plan and deliver the therapy, or a combination of both.

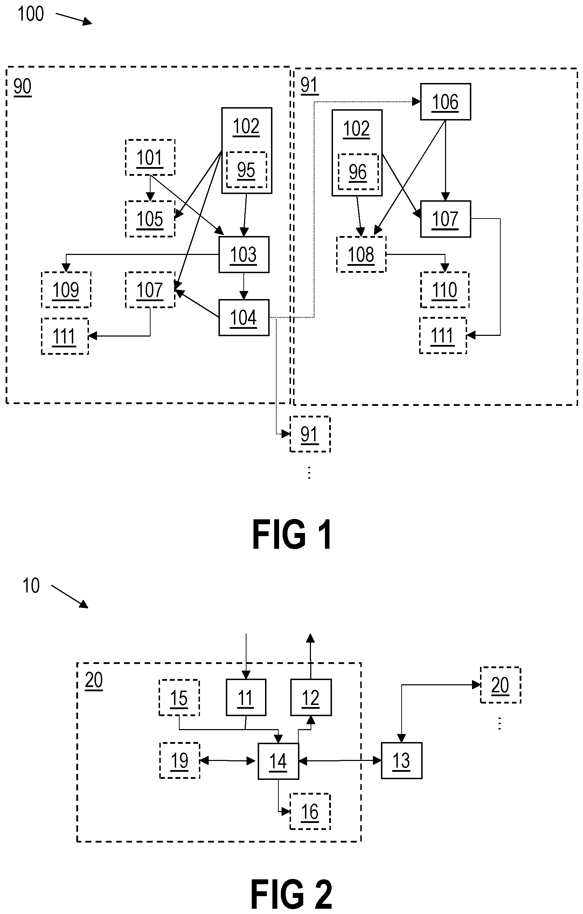

Referring to Fig. 1, an illustrative method 100 in accordance with embodiments of the present invention is shown. The method may comprise, for a first session of a multisession series for a same patient, selecting 101 a breathing goal from a plurality of different breathing goals. The selection may comprise a selection from a plurality of discrete possibilities, and/or a selection of a parameter or combination of parameters from (a) corresponding continuous parameter range(s) (or a stepwise approximation thereof, e.g. on an ordinal scale representing steps of the parameter value). Alternatively, the method may be adapted to aim for a predetermined, fixed (high-level and/or generic) breathing goal, e.g. such that no selection is strictly necessary. Nonetheless, it can be assumed that a breathing goal is available, either being predetermined (e.g. fixed) or after a selection 101 from various possibilities.

The selection 101 of the breathing goal may relate to defining a target for one or more breathing parameters (e.g. selecting the relevant parameter or parameters from a plurality of options indicating different types of parameters, and/or selecting a range and/or lower threshold and/or upper threshold for a value of the parameter or parameters). Thus, the patient's breathing pattern can be influenced, as discussed further hereinbelow, by performing the method in order to meet the selected breathing goal, e.g. decrease or increase the breathing parameter, to maintain the breathing parameter in a target range, or combinations thereof for a plurality of different breathing parameters. Examples of breathing parameters include an amplitude (e.g. of lung volume or a correlate thereof, e.g. a chest movement indicator), a maximum inspiration level, a maximum exhalation level (or, in other words, a minimal lung volume of a breathing cycle), a breathing frequency, a mean or other measure of statistical centrality of any of the aforementioned parameters over a plurality of breathing cycles, a variance or other measure of statistical dispersion of any of the aforementioned parameters over a plurality of breathing cycles, and/or any other parameters that can characterize an aspect or aspects of the breathing pattern.

Thus, embodiments of the present invention may aim to create a situation in which a patient has substantially a same (in good approximation) breathing pattern during different sessions, e.g. imaging on various imaging modalities (e.g. CT, MRI, and/or other imaging modalities) and/or during treatment, which may equally consist of multiple treatment sessions. Particularly, the targeted breathing pattern can be configured as function of a global (e.g. high-level) goal for the entire series of sessions, i.e. the selected goal is not necessarily optimal for one of the sessions in isolation, not even for the first session where it is applied. For example, the first session may be an imaging session, e.g. an MRI scan, but the breathing goal may be selected as likely being optimal (or at least particularly suitable) for a treatment of a specific disease in a later session (e.g. radiotherapy for treating lung tumor nodules).

The step of (for a first session) selecting 101 a breathing goal may comprise receiving input from an operator (generally a healthcare professional or user of a system or computer program product implementing the method) to select the breathing goal from a (discrete and/or continuous) plurality of supported breathing goal options. For example, the operator may enter a selection via a non-interactive or interactive interface, e.g. a graphical user interface. This selection may comprise one or more choices, which may include selection(s) from list(s) of discrete options and/or selection(s) of continuous or ordinal values (e.g. thresholds, ranges and the like for comparing a parameter value against). Receiving the input may also comprise retrieving the input from a storage medium or remote system, e.g. where the selection was stored prior to the first session by a healthcare professional planning the multisession series and/or from a remote terminal of a staff member supervising the session(s).

For example, when it is the first time the patient is imaged (at least for the specific series of imaging and/or treatment sessions that are unified in their global intended outcome or purpose), the healthcare professional can set the breathing behavior to capture and promote by influencing the patient's breathing behavior.

The method comprises monitoring 102 the respiration of the subject by (e.g. continuously, or at least frequently) measuring, using a sensor, one or more respiratory parameters. Generally, the sensor refers to any suitable sensing device, i.e. a device or system capable of determining a relevant parameter that can characterize an aspect of the respiration from an observed physical quantity or quantities. The respiratory parameter may be a parameter that is clearly directly related to respiration, e.g. air volume in the lungs, in/exhaled air flow and/or breathing frequency, and/or that is sufficiently related to and/or indicative of (a) characteristic(s) of the respiration cycle, e.g. changes of a marked position on the chest due to breathing, measurements of expansion of the chest and/or relevant information derived from camera observation images (e.g. a video stream) by image processing techniques. This also implies that the sensor or sensing device can take many forms, e.g. may comprise a spirometer, a proximity and/or ranging sensor, a radar sensor or similar technology (lidar, sonar, ...), a pressure sensor or array of pressure sensors, and/or a camera or combination of cameras (e.g. arranged for depth imaging), without limitation to these examples. When the sensor or sensing device comprises one or more cameras, these can comprise a monochrome camera, a color image camera, and/or a multispectral camera, and/or can operate in the (human) visible spectrum, the infrared spectrum, a combination thereof, or a small sub-band thereof (e.g. only a specific color component). This also means that the at least one respiratory parameter that is measured may be derived from the data provided by the sensor or sensing device in a straightforward manner (e.g. the sensor may provide an output that is directly usable as a respiratory parameter of interest) or may require some processing, e.g. to determine a robust parameter (e.g. correlated with or at least indicative of a current air volume in the lungs, a chest expansion level, a breathing frequency, ...) from the data, e.g. from a video stream. However, many suitable methods are known in the art to determine one or more respiratory parameters from various data sources (including, for example, video camera observation), and can thus be readily implemented in embodiments in accordance with the present invention if needed.

Since the method relies on a (e.g. "live" or "real-time") monitoring 102 of the respiration, in each of the sessions of the multi-session series, and different sessions may be performed in different settings, e.g. in a different room or theatre, using different equipment (e.g. scanners, therapy systems), with the patient positioned on a different couch and/or, generally, under different conditions, the method may comprise acquiring sensor data from different sensors (which may also include, for example, camera observation systems from which relevant information can be derived) in different sessions of the multisession series, and determining the (same) one or more respiratory parameters from the acquired sensor data from different sensors. In other words, the same one or more respiratory parameters are determined in different sessions, even if based on different sensors or even different types of sensor. Thus, regardless of the sensor (e.g. using a specific sensor calibration) or even type of sensor, e.g. regardless of sensor modality, the acquired signal(s) may be transformed and/or converted such as to deduce a same measure or measures indicative of breathing across the entire multi-session series. For example, in one session, a respiratory belt sensor may acquire a signal indicative of a pressure difference, whereas in a different session, a camera may acquire images in which movement of the chest is observed. Dedicated processing algorithms may be used to deduce from each such sensor modality a same parameter indicative of breathing, e.g. a measure (e.g. point-measure in time) indicative of a current air volume filling the lungs and/or another generalized indicator of breathing, e.g. a breathing velocity, a breathing rate, a distance between reference points where at least one point is subject to motion due to breathing, .... This is schematically illustrated in Fig. 1 by the optional sensor-specific conversion steps 95,96 to generate the at least one respiratory parameter from different sensor modalities in different sessions.

The method comprises, for a first session of a multisession series for a same patient, determining 103 when a breathing goal, i.e. the selected 101 breathing goal or a predetermined breathing goal, has been reached, taking the monitoring 102 of the respiration of the subject into account. In other words, the measured respiratory parameter(s) may be (e.g. in real-time) analyzed to determine when the breathing goal has been reached (e.g. to a sufficient extent).

In a particularly simple embodiment, an operator may observe the respiratory parameter(s) or an analysis thereof (e.g. a live feed showing a continually updated representation of the current breathing behavior), may decide when the goal has been reached and then indicate so via a user interface.

In a more intricate embodiment, the observed respiratory parameter(s) may be tested against a criterium or criteria representative of the predetermined or selected goal. The criterium or criteria may be as simple as a target threshold(s) or range(s) for a respiratory parameter or parameters (e.g. directly observed by the sensor or sensors, and/or derived from the collected sensor data), or may take a dynamic change of such parameter(s) into account. For example, as will be explained further below, the patient may be coached toward the goal, which will be reflected by changes in one or more respiratory parameters toward a desirable state. Similar to numerical optimization methods, the criterium may take a rate of change of the relevant parameter or parameters into account to determine when an optimum has been reached with negligible further (diminishing) returns. This means that the criterium for deciding when the goal has been reached can be easily adapted to what is achievable for a specific patient, e.g. may merely require that a parameter should be maximized or minimized up to the limit of what is comfortable and/or achievable by the patient without requiring a hard threshold on the relevant parameter(s).

It is also noted that an automated approach to determine 103 when the breathing goal has been reached does not preclude the option of the operator interacting with the automated process, e.g. to override the conditional testing loop when he/she deems no further progress toward the goal is likely. In other words, an automated process to determine 103 when the breathing goal has been reached, based on a stream of sensor measurements (or variable or variables derived therefrom), can be combined with a manual decision process by an operator, e.g. receiving input from the operator to indicate when the goal has been reached.

The first session may typically be an imaging session intended for acquiring image information to plan a future therapy session in said multisession series. This means that the operator may also take imaging information into account if needed. For example, low dose CT scans may be taken during the phase of determining when the breathing goal has been reached, or, similarly, a sequence of MRI images may be taken at a sufficiently high temporal resolution (e.g. using echo planar imaging, without limitation thereto), such that the operator can take an observed movement of a target feature in the body into account. For example, the operator can observe movement in a region to be treated, and decide when the breathing goal has been sufficiently reached without adversely affecting the movement in the target region of the body.

An example would be a case where the selected or preset goal is a shallow breathing pattern. A criterium may be automatically monitored to decide when the goal has been reached and/or the operator may directly observe a live feed representative of the breathing behavior and may manually decide when a sufficiently shallow breathing pattern has been reached. The automatic criterion could be, for example, a breath amplitude below a predetermined threshold, or (an absolute value of) a change rate (change per unit of time) of the amplitude below an absolute or relative (e.g. percentual change) threshold indicating that no substantial further progress toward the minimum amplitude is being made. However, in accordance with some embodiments of the present invention, the operator may also take imaging information into account to confirm that a region in the patient's body, e.g. where a tumor to be treated is located, is not moving too much, and accordingly override the decision of the automated procedure if needed, or take this additional information into account in his/her manual decision.

Examples of such goal behavior may be a slow breathing, a shallow breathing or breathing with an extended end exhalation. It is an advantage that a breathing pattern of a specific patient, within his/her capabilities, can be characterized to be reproduced later, e.g. by storing parameters indicative of the observed breathing pattern. Additionally, other parameters which are associated with the feedback applied to obtain the desired breathing pattern may also be stored, such as a speed at which the coach changed the behavior. This allows for an efficient reproduction of the same breathing pattern in later sessions, and/or a more accurate planning of the required time for a session.

The method further comprises, for a first session of a multisession series for a same patient, determining 104 a reference breathing pattern characterization from the respiratory parameter(s) once it is determined 103 that the predetermined or selected breathing goal has been reached, and storing the reference breathing pattern characterization for future use.

Furthermore, in a method in accordance with embodiments of the present invention, a trigger may be generated 109 once (i.e. when) it is determined 103 that the predetermined or selected breathing goal has been reached, to provide a signal to an imaging or therapeutical system so as to commence with a scanning or therapy operation.

The method may also comprise providing 105 feedback to the subject to influence the subject's breathing while monitoring 102 the respiration in order to meet or approximate the predetermined or selected breathing goal. Thus, until the breathing goal is determined 103 to have been reached, the subject can be actively guided toward a breathing pattern that corresponds to the goal by monitoring 102 the respiration and providing a feedback signal to the subject, e.g. by comparing the measured respiratory parameter(s) to a target defined by the goal and presenting a feedback to the subject that represents the currently observed parameter(s) contrasted with the target, a difference or other type of deviation measure between the current and target values, or in another way to enable the subject to gain insight in the alignment of his/her present breathing pattern with the predefined or selected goal. It will be understood that the subject can thus be provided with information on his/her present breathing and the intended goal, such that the subject can evaluate his/her performance in substantially real-time, e.g. by a mental comparison, but that such comparison may also be performed as part of the method in accordance with embodiments, e.g. by an automated computation. Thus, the subject may be presented with a measure or indicator (which may, generally, be in the form of any sensory signal or combination of such sensory signals) of his/her breathing performance as determined by such automated process.

The feedback 105 may also continue, after the reference breathing pattern has been determined and/or the trigger has been generated 109, such as to assist in maintaining the same breathing pattern to reproduce.

Such coaching can take various forms and shapes, potentially based on multi-modality input, e.g. different types of sensor input and/or different measured or derived parameters representative of aspects of breathing. A typical example is displaying the desired breathing pattern and providing insight or feedback on the accuracy of the actual breathing pattern. For the latter, the method may check in how far the actual behavior is near to the desired goal behavior to provide suitable feedback, which may also be used to automatically determine 103 that the breathing goal has been reached and hence trigger the determination 104 and storage of a reference pattern. For example, the observed breathing behavior may be first coached towards a desired behavior optimized for imaging, e.g. shallower breathing, which can then be stored as the breathing target for future scans.

The step of providing feedback may furthermore continue after the goal has been (adequately) reached, e.g. by the step of determining 103 when the breathing goal has been reached, to support the subject to continue this breathing goal throughout the session by means of coaching.

However, preferably, the method may use the reference behavior as a new target for the feedback mechanism after it has been determined 104. This is similar to what is discussed further hereinbelow in the step of providing 107 feedback to the subject during a second or further session of the series to influence the subject's breathing while monitoring 102 the respiration in order to match or approximate the earlier stored reference breathing pattern characterization by taking the monitored 102 respiratory parameter(s) of the subject into account.

In embodiments according to the present invention, the method may, in the first session after the reference breathing pattern has been determined 103 and/or in (a) subsequent session(s), continue to aim for gradual improvements toward the initial goal and update the reference behavior for future sessions if substantial improvements are made toward the initial goal relative to the determined reference behavior.

However, keeping the reference behavior fixed (after the initial trigger by the step of determining 103 that the breathing goal has been reached) can have advantages in terms of maximizing the reproducibility. On the other hand, under some circumstances, further training toward the goal and gradually updating the reference may have advantages as well, e.g. to ensure that a goal optimized for a treatment protocol is instilled as learned behavior in the subject throughout the multisession series.

Nonetheless, by storing the reference behavior determined from actual performance of the subject, even if gradually updated, it is ensured that each future session can start from a reference target that has already been achieved by the subject in an earlier session, thus smoothening the learning curve. It is also noted that, in an embodiment, updating of the reference breathing pattern characterization may be configurable, e.g. an operator or other clinical worker may select whether the advantage of further training toward the initial goal outweighs the advantage of a higher reproducibility of the breathing pattern.

As example, WO 2020/069948 A1 discloses a suitable method for influencing a breathing parameter of the subject's breathing pattern in order to meet the goal. In this illustrative approach, the breathing parameter can be decreased or increased (toward a target in line with the goal) by monitoring a current value of the breathing parameter and providing sensory feedback to the subject about a determined preferred value (different from the current value) of the breathing parameter for achieving the goal. For example, the preferred value of the breathing parameter may be determined by means of an artificial intelligence intelligent agent, as described in the referenced publication. However, other approaches for coaching the breathing pattern toward a specific goal are also known in the art and not necessarily excluded.

It is an advantage that an active coaching approach (i.e. providing 105 the feedback) may typically use a measure of correspondence between the currently observed parameter(s) and target(s) representative of the goal, such that it can be easily determined from this measure of correspondence (e.g. a reward function in the cited prior art example) if the goal has been sufficiently reached, at which point the method can determine 104 the reference breathing pattern.

For example, the reference breathing pattern characterization may comprise an average (or other measure for aggregating over a plurality of breathing cycles; e.g. a measure of statistical centrality) of the respiratory parameter or parameters over a plurality of breathing cycles, e.g. as measured when or (e.g. shortly) after the breathing goal was determined to be reached. The respiratory parameter or parameters used to determine the reference breathing pattern may be the same as used for testing the breathing goal criterium/criteria, but this is not necessarily the case. The reference breathing pattern may thus comprise one or more of the following (not limitative examples): a breath rate (or period, frequency, ..., e.g. breaths per minute), a breath amplitude (e.g. difference between measures characterizing maximum inhalation and maximum exhalation, e.g. difference in volume, chest circumference, position of a reference point on the chest, etc.), a variability (e.g. standard deviation or other measure of dispersion) of breathing rate and/or amplitude, a value representing average maximum inhalation over the observed breathing cycles (e.g. a lung volume, chest circumference, maximum chest elevation of a reference point along anteroposterior axis, ...), a value representing average maximum exhalation over the observed breathing cycles, a value representing the average lung volume (averaged over multiple cycles, alternatively of other variable such as chest elevation or circumference, ...), an (average) inhalation duration, an (average) exhalation duration, and/or a timeseries representing an average breathing cycle (of a relevant parameter as discussed, e.g. volume, circumference, AP displacement, ..., e.g. such variable as function of time over a single cycle by averaging or otherwise aggregating multiple cycles). The reference breathing pattern characterization may also comprise one or more values related to breath holds, e.g. a (average) breath hold duration (or capacity), breath hold amplitude or position (again, a positional measure such as a distance to a reference point on the chest, an air volume in the lungs, a chest circumference measurement, or any other suitable measure to characterize the breath hold point in the breathing cycle). Such breath hold related parameter(s) may be particularly relevant (either alone or in combination with another or other measures as mentioned hereabove for a more complete characterization) for a predetermined or selected goal that involves holding breath repeatedly, e.g. mini breath holds, but are not necessarily limited thereto.

However, the breathing pattern characterization is not necessarily limited to measuring or deriving one or more parameters indicative of the breathing pattern. For example, instead of directly storing a characterization of the patient-specific 'natural' (or at least 'coached') breathing goal, a database may be referenced that stores reference breathing behaviors to select a pattern from the database that matches the observed pattern (e.g. a best approximation thereof). Thus, an option can be selected from the database that comes close(st) to the observed breathing behavior, such that this pre-determined breathing pattern from the database can be reproduced in further sessions. This has the advantage that a specialized feedback method (or configuration) can be used for the specific breathing pattern. For example, each pattern stored in the database can be associated with a specific approach to coaching a subject to reproduce this pattern. Alternatively or additionally, a therapy planning system can be adapted to take such specific reference behaviors into account in defining a treatment plan.

Thus, to summarize the aforementioned, a method in accordance with embodiments, e.g. as may be implemented in a "breathing coach" software (without limitation thereto), can be applied after a subject has been prepared for a first imaging or therapy session, e.g. positioned on an imaging or therapy couch. When the patient is positioned on the table and a respiratory signal is received from a respiratory sensor (in a broad sense, e.g. possibly including signal or signals representative of respiration that are in substantially real-time derived from camera observation or other types of sensor data), the respiratory signal(s) can be analyzed (e.g. by the breathing coach software). The method is configured for a predetermined goal, or a suitable goal is selected from a plurality of options (e.g. from discrete configurations and/or from a continuous range, or from a combination thereof). The method determines when the goal is (sufficiently) reached based on the analysis, or the analysis is presented (preferably in substantially real-time and preferably with substantially continuous updates) to the operator in order to relegate the decision when the goal is reached to the operator based on the presented analysis data. As mentioned, a combination is also possible, e.g. a semi-automated or supervised process in which an operator can override or otherwise interact with the automated process. Once the goal is deemed sufficiently reached, the method (e.g. the breathing coach software) determines the reference breathing pattern characterization (e.g. the relevant current average breathing behavior), e.g. based on the respiratory signal(s) collected over multiple breathing cycles. When the reference breathing behavior is determined, it is saved as the breathing behavior target for future imaging and/or therapy sessions.

It is noted, however, that the first session may alternatively be split into a preparatory phase and the imaging or therapy session as such. For example, the initial steps of determining 103 when the predetermined or selected breathing goal has been reached based on the monitored respiration and determining 104 and storing the reference breathing pattern characterization once that the breathing goal has been reached may be performed in a different environment than used for the imaging or therapy session as such, such as a waiting room, a preparation room or even at home before checking into the medical facility for the imaging or therapy. This may also include scenarios where the first session is, for example, merely a simulation of an imaging or therapy session to be performed later, solely for the purposes of training and characterizing the breathing behavior that is expected, or at least desirable, in that future session. It is also an advantage that, at least for some embodiments, the breathing goal is not predefined, but rather selectable/customizable as function of the intended purpose. It is furthermore an advantage that this goal is not necessarily optimal for the session in which it is first used and/or selected, but can be selected as optimal for a future session in the series. It is also an advantage that embodiments of the present invention aim to create a high degree of reproducibility across session in the series, e.g. aim to produce the same breathing behavior in each session.

It is an advantage of embodiments of the present invention that the breathing goal is not merely selected and stored for future use, but instead a characterization of the breathing pattern that is achieved by the patient in line with this goal is stored to be reproduced in later sessions. For example, instead of aiming in each session to achieve or optimize breathing in line with a particular breathing goal, an initial phase of the first session is used to coach the patient toward the goal, and later sessions try to reproduce the breathing pattern thus achieved instead of separately aiming to achieve the goal. Thus, reproducibility can be given a higher priority than actual compliance with the goal, while still achieving the goal to some extent.

For example, the level of anxiety (or other mental factors influencing compliance and/or abilities of the patient) of the patient can vary in different sessions and thus affect the performance when only the goal as such were to be considered. Another factor that could affect the breathing performance could be a (light) sedation which might be applied in some sessions while not in others. Furthermore, as the number of past sessions (imaging or therapeutic) increases, the patient may have experienced repeated training events in the breathing paradigm, such that better results in line with the goal could be achieved if only considering the goal as such. Nonetheless, by storing a characterization of the initial breathing pattern that was achieved during the first session, and aiming in later sessions to reproduce this initial breathing pattern instead of the goal that was initially targeted, reproducibility across imaging and/or therapeutical sessions can be improved (with the advantages associated therewith), without risking variation due to better or worse performance in achieving a more generic goal. Furthermore, it is ensured that the pattern which the patient is expected to reproduce (in follow-up sessions) is actual achievable by the specific patient, since it was actually realized by the patient in the first session.

To illustrate embodiments of the present invention, in a first (e.g. imaging) session, e.g. a CT scan, a healthcare professional (e.g. an operator) may position the patient (to which the multisession series relates) on a couch (i.e. the examination table top). At this stage, one or more respiratory sensors and/or feedback devices may be set up (e.g. to emit a signal perceivable by the patient, e.g. a screen, a vibrating element, an actuator, headphones, a loudspeaker, etc.). For example, a patient-facing display and/or actuator may be used to provide feedback to the patient. However, it is to be noted that breathing behavior can also be deduced from, for example, remote camera observation of the patient (and suitable video processing) and/or feedback can be provided by information presented on a monitor or projection screen, such that little or no setup may be required for the feedback system. The healthcare professional can then configure the imaging procedure, e.g. set up a 4D CT procedure via a user interface of the CT scanner. Additionally, using a user interface of a system implementing the method in accordance with embodiments, e.g. a breathing coach software, the healthcare professional can configure a high-level breathing goal, e.g. shallow breathing, regular breathing, a breath hold duration and/or target point of the breath hold in the breathing cycle, combinations thereof, or, generally, any breathing goal that would be desirable for the multisession series as a whole (e.g. taking a planned future therapeutic session into account). It will be clear that the user interface of the breathing coach system may also be integrated in the user interface of the imaging or treatment device, or may run on a same computer system.

Once activated, the breathing coach starts to measure one or more respiratory signals of the patient. Based on this respiratory information and given the high level-goal, the breathing coach determines the average breathing pattern of the patient, i.e. once the goal is determined to be sufficiently reached (automatically, manually by interaction by the healthcare professional, or a combination thereof).

Given that this is the first scan and no reference breathing pattern to reproduce is yet available, the breathing coach stores this average behavior, e.g. centrally in a database or other suitable storage where it can be easily accessed for future sessions even if not on the same system, e.g. the same CT scanner. At this stage, i.e. once the high-level goal has been sufficiently reached and the reference breathing information is determined, a trigger may be generated to signal to the imaging system to commence the scanning operation. The breathing coach may furthermore continue to coach the patient to keep up with the exact (within a reasonable approximation) behavior as represented by the reference throughout the scan. For example, the target criteria of the initial feedback implementation (see step 102) may be adjusted to the reference breathing pattern characterization as determined (or may be replaced by a feedback method that is more suitable for matching the reference pattern as opposed to the high-level goal). Thus, once the reference is established, the breathing coach may continue to analyze whether the patient's breathing behavior is coherent with the reference pattern as earlier determined and correct deviation by coaching the patient toward the desired behavior (i.e. the reference determined for the patient) to achieve a good consistency and reproducibility throughout the scanning operation (as well as in future sessions, as discussed below). Meanwhile, the healthcare professional may be presented with information to monitor the compliance of the patient with the reference breathing pattern, such that he/she can intervene when deemed necessary, e.g. pause or stop the scanner and recommence or repeat the operation when a good matching behavior is again observed (e.g. possibly after personal interaction with the patient).

Thus, the method (e.g. implemented in a breathing coach software) can be used to create a situation in which the patient will execute similar breathing behavior on various imaging and/or treatment modalities. For example, in a first session, CT images may be acquired, while in a next session, MR images may be acquired under the same breathing conditions. Thus, a reproducible (and/or regular) breathing pattern is obtained, such that different images can be better correlated. This may be followed by a free-breathing treatment, or the same approach may be extended to also ensure that the same breathing pattern is repeated during treatment.

Alternatively, the method may be implemented by software and/or hardware (generically referred to as the "breathing coach") in such way that manual interaction is reduced or minimized. For example, the start of the breathing coach, e.g. of the step of monitoring 102, may be automatically triggered by an instruction to the scanner system to commence the selected protocol or, generally, may be controlled by the imaging system. Likewise, the high-level goal may be predetermined or may be automatically selected by (e.g. deduced from) the selected scan protocol and/or type and/or (a) selected configuration parameter(s) of the scan protocol. As already mentioned, the start of the actual imaging operation can also be activated by a trigger from the breathing coach, without requiring manual interaction from the healthcare professional. Also, pause and resume of the scan operation may be substantially automated by further triggers from the breathing coach by taking a deviation (not to be construed in a limitative way, e.g. generally any suitable measure that indicates a degree of correspondence or deviation therefrom) into account during active monitoring and feedback after start of the operation, e.g. such as to pause the scanning when the breathing behavior temporary fails to match the reference behavior. For example, a pause signal 111 may be generated to interrupt the scanning or therapy operation when the subject's breathing deviates from the reference breathing pattern (e.g. deviates more than a dynamic and/or fixed threshold, and/or when a criterium representative of a significant deviation is met).

A functional difference between coaching the breathing behavior toward a high-level goal and toward matching a stored reference behavior (optimized for such goal) is emphasized. For example, a high-level goal may be sufficiently matched by different breathing patterns, whereas different breathing behaviors in line with such high-level goal are not necessarily sufficient to ensure a high degree of reproducibility and/or regularity within and across sessions. For example, if the high-level goal corresponds to a desirable "shallow breathing" pattern, which may be encoded by a respiration amplitude below a certain threshold, different breathing behaviors may satisfy this high-level criterium while in poor correspondence with each other, e.g. showing a large difference in the average air volume in the lungs around which the respiration cycle is centered or being below the threshold but possibly still differing substantially in amplitude (in relative terms with respect to each other).

The illustrative method 100 in accordance with embodiments of the present invention further comprises, in a second and (if included in the series) each further (third, fourth, ...) session of the multisession series for the same subject, retrieving 106 the stored reference breathing pattern, as determined in a previous (e.g. the first) session.

The multisession may also comprise, in addition to the imaging and/or therapy session(s), (a) dedicated breathing training and/or simulation session(s). For example, the breathing behavior may be practiced by the subject in a training or simulation session prior to an actual imaging or therapy session. For example, the first session may be such training session or a simulation session (e.g. simulating a future imaging or therapy session of the series), in which the reference breathing pattern is determined for future use. Likewise, one or more training sessions may be included between, for example, imaging and/or therapy sessions, to ensure that the patient is, and remains, sufficiently familiar with reproducing the intended breathing pattern.

As mentioned hereinabove, the method comprises monitoring 102 the respiration of the subject by measuring, using a sensor or sensing device, one or more respiratory parameters, which applies equally to each of the sessions of the multisession series.

Furthermore, the method comprises, in the second and further (if included) sessions of the multisession series, providing 107 feedback to the subject to influence the subject's breathing while monitoring 102 the respiration in order to match or approximate the earlier stored reference breathing pattern characterization (e.g. determined 104 and stored in the first session, or, after that, an updated version thereof), by taking the (presently) monitored 102 respiratory parameter(s) of the subject into account.

The method may also comprise, for the second and/or further session (if included in series), determining 108 when the earlier stored reference breathing pattern is matched or sufficiently approximated (e.g. considering a measure of deviation, which may e.g. be compared to a static or dynamic, e.g. absolute or relative, threshold). In other words the measured respiratory parameter(s) may be (e.g. in real-time) analyzed to determine when the breathing characteristic(s) determined in an earlier session has/have been matched (e.g. to a sufficient extent).

Furthermore, a further trigger may be generated 110 once (i.e. when) it is determined 108 that the reference breathing pattern is matched or sufficiently approximated by the subject's breathing, to provide a signal to an imaging or therapeutical system so as to commence with a scanning or therapy operation.

The feedback 107 may also continue, after determining the reference breathing pattern has been matched and/or generating 110 the trigger, such as to assist in maintaining the breathing pattern to reproduce.

Thus, the subject can be actively guided toward a breathing pattern that corresponds to the stored reference breathing pattern characterization by monitoring 102 the respiration and providing a feedback signal to the subject. Such coaching can take various forms and shapes, potentially based on multi-modality input, e.g. different types of sensor input and/or different measured or derived parameters representative of aspects of breathing. The coaching (feedback) method step targeting reproduction of the reference may be the same as the initial coaching method step targeting the global goal in the first session, after suitable reconfiguration (e.g. setting appropriate parameters), or may be different.

As already mentioned for the initial coaching method step, a typical example may be displaying the desired breathing pattern (here corresponding to the stored reference) and providing insight or feedback on the accuracy of the actual breathing pattern. Thus, the method may check in how far the actual behavior is near to the stored reference behavior to provide suitable feedback. Reference is made again to the example disclosed in WO 2020/069948 A1, but many examples can be found in the art that are potentially suitable.

Thus, the second and further times the patient is imaged and/or treated (at least, for the same purpose and/or intended therapeutic outcome), the breathing coach can retrieve the stored breathing pattern, and coach the patient's breathing to this desired breathing pattern. This feedback can be as crude as simply visualizing the desired breathing pattern in the form of a breathing pattern or breath hold depth, e.g. in combination with a visualization of the current breathing as determined by the monitoring. More sophisticated approaches may gradually guide the patient from his/her current breathing pattern to the desired one.

The visualization may be simple numerical or graphical representation of the target values (preferably in combination with corresponding values of the current performance) and/or of an indicator that represents the present performance for the desired target breathing pattern, and/or the feedback may use other sensory modalities, e.g. providing audio feedback (e.g. a synthesized voice giving instructions and/or similar information as can be displayed or a tone varying in height or amplitude to represent correspondence or deviation of the behavior versus the target, without limitation to these examples), a vibration or other tactile signal, and/or another suitable biofeedback means. When the patient's breathing behavior matches the goal behavior the imaging or therapy system may be triggered, e.g. activated, to perform the image sequence or deliver the therapy, e.g. deliver a therapeutic radiation dose.

In an embodiment of the present invention, the feedback method (e.g. in steps 105 and/or 107, in the first and/or further sessions) may be configurable, such as to adapt the feedback mechanism to the specific subject and/or conditions at hand. For example, a feedback modality may be selected based on preference of the subject and/or operator, and/or on technical capabilities of the system available in the imaging/treatment room, and/or on capabilities of the subject (e.g. visual only, audio only, a combination of visual and audio, ...). Not only the sensory modality of the feedback can be varied (e.g. may be selectable), but options may be provided as to the specific form, e.g. from a more technical visualization to a simpler visualization scheme suitable for children. Another factor that may be provided in selectable options could relate to a specific color scheme for visualizations, such as to integrate in a specific imaging/treatment room for a specific session and/to accommodate a color vision deficiency of the subject. The selection may be stored to reuse in further sessions, or to reuse in part (e.g. in so far possible, e.g. taking further technical constraints of the setting in a different session into account as well). This allows a similar or the same experience to be presented to the subject in further sessions, which may aid in reproducing the breathing pattern accurately and/or to put the patient at ease.

Returning to the example discussed hereinabove, after an initial CT imaging session, an MRI session may be performed as a follow-up scan. Again, the patient may be positioned appropriately for the imaging procedure, e.g. in a different room and/or imaging system than used for the first session. This time, the breathing coach software can retrieve a characterization of the breathing pattern as obtained in the first imaging session, which can be reproduced in the present session by coaching the patient toward this target. When it is detected that the subject is compliant with the earlier established pattern, the MR system's scanning protocol can be activated, e.g. a 4D imaging protocol. This can be triggered automatically, or by interaction with a supervising healthcare professional. When it is observed that the patient's behavior is off, the healthcare profession may also intervene, e.g. by pausing the procedure automatically or manually. For example, when the breathing is slightly off target, the healthcare professional may interact with the patient and stimulate the patient to follow the pattern, and/or when severely off, the scanning procedure may be paused until the desired breathing behavior is again established.

After the imaging sessions, the acquired images, e.g. CT and MRI, may be registered. The registration procedure (e.g. a rigid and/or non-rigid transformation and/or deformation such as to minimize differences), a treatment plan may be determined based on the imaging information. It is an advantage that in this treatment planning, the expected motion from the established reproducible breathing pattern can be taken into account. For example, a treatment may be scheduled on a suitable treatment device, e.g. a combined MRI and radiotherapy system (MR-Linac). Optionally, the same breathing guidance approach may be used in this therapy (or combined therapy/imaging) session, such as to reduce deviation from the established breathing pattern, which may be particularly advantageous when the treatment was planned by implicitly or explicitly taking this breathing pattern into account as additional planning information. For example, after setting up the patient and the treatment on the therapy system, the breathing coach software may be activated to coach the patient to reproduce the earlier established breathing pattern. When reached, the treatment system may be activated, and can optionally also be paused if needed (e.g. when a substantial deviation from the stored reference breathing pattern is detected).

Treatments, such as radiotherapy, may be performed in multiple sessions, e.g. multiple radiotherapy fractions. Therefore, in every therapy session, the same breathing guidance approach may be applied to coach the patient in executing the same breathing behavior. Thus, a reproducible location range of a moving target of interest for the therapy may be obtained across the therapy sessions. Since the same breathing pattern was followed for acquiring the imaging data used in planning the procedure, an accurate planning can be obtained. Furthermore, if the therapy session(s) relies on live imaging, the pre-acquired images can serve as a good basis to compare the live imaging information against to detect and/or prevent (or reduce) deviations from the target in the therapy delivery.

In a second aspect, the present invention relates to a computer-program product for performing, when executed on a computer, a method in accordance with the first aspect of the present invention.

In a third aspect, the present invention relates to a device for providing breathing guidance to a subject during multiple diagnostic imaging and/or therapy sessions of a multisession series for said subject. For example, the device may be adapted for performing a method in accordance with embodiments of the first aspect of the present invention.

Fig. 2 illustrates an illustrative device 10 in accordance with embodiments of the present invention.

The device 10 comprises an input 11 for receiving sensor data and determining at least one respiratory parameter therefrom to continually monitor the respiration of the subject during each of said sessions. The input may comprise a sensor or plurality of sensors (e.g. a spirometer, a camera, and/or another sensing device suitable for acquiring information from which the respiration of the subject can be characterized), and/or is adapted to be operably connected to such sensor(s). Examples of suitable sensors and parameters to be derived therefrom were discussed hereinabove, without limitation to these examples.

The input 11 may also comprise a converter 17, such that the same at least one respiratory parameter can be determined from the signal(s) obtained from different sensors. For example, the converter may be adapted to detect (or may be configurable for) different types of sensor and apply suitable processing to determine the same respiratory parameter(s), regardless of the specific type of sensor input that is received. As another example, different embodiments of the device 10 may be outfitted with different converter modules (which may be implemented in hardware, in software or by a combination of both), such that these different devices can be used together (e.g. in different sessions of a same multisession series for a specific patient), each determining the same respiratory parameter(s), when in use, based on inputs received from different types of sensor for each specific device.

The device 10 also comprises a feedback output 12 for providing sensory feedback to the subject in each of said sessions. The sensory feedback may be visual, auditive, tactile and/or delivered by another sensory feedback mechanism to the subject, or a combination of different sensory modalities. Therefore, the feedback output may comprise a monitor, a projector, an indicator light, headphones, a speaker, an actuator, a vibrating element, and/or another suitable device for delivering sensory feedback, or a combination of means for delivering different sensory modalities. Alternatively, the output may be adapted to operably connect to such output device(s).

The device 10 comprises a data storage unit 13 for storing data, e.g. the reference breathing pattern characterization. For example, the data storage unit may comprise a data storage disk, a hard drive, a database, a network drive or similar means for storing digital information.

The device 10 also comprises a processor 14. For example, the device may comprise a computer, an application specific integrated circuit and/or configurable digital processing hardware for performing the processing tasks discussed hereinbelow, e.g. method steps of a method in accordance with the first aspect of the present invention as discussed hereinabove.

The device may also comprise a user interface 19 for interacting with an operator, e.g. a healthcare professional, such as a medical doctor, a nurse, a radiotherapist, an imaging technologist or the like. For example, the user interface may be used by the operator to control and/or monitor the breathing guidance provided by the device.

The processor 14 may be adapted to determine whether a current session corresponds to a first session or a further session of the multisession series. This can be, for example, achieved by checking whether the reference breathing pattern characterization for a specific series is already stored in the data storage unit 13, by receiving input from the operator via the user interface 19, and/or via another input, e.g. a scanner or therapy system interface, e.g. by retrieving the state of an indicator flag in a scheduling system.

In accordance with some embodiments, the device may comprise multiple separate units 20, each comprising an instance of the input 11, the feedback output 12 and the processor 14 (and optionally other features, such as the trigger signal output 16 and user interface 19 as well). Each instance may be essentially identical, e.g. capable of performing the same functions. Alternatively, one instance may be adapted for being used for the first session of a multisession series, while one or more instances may be adapted for being used for the further session(s) of the series. In other words, the evaluation as to whether a session corresponds to the first session may be determined by the unit module that is applied, e.g. an sequence initiator software package or a sequence follow-up software package.

The plurality of units 20, e.g. each unit, may be operably connected to the (same) data storage unit 13, such that different units have access to the same reference breathing pattern characterization, e.g. such that different sessions of the same series can be performed by different units. Thus, the units may respectively operate in different rooms and/or in combination with different imaging and/or therapy systems to provide breathing guidance for different sessions of the multisession series.

Furthermore, at least some units may comprise, as part of the input 11, a converter 17 for receiving sensor input from respectively different sensors (e.g. having different calibrations associated therewith and/or even of essentially different sensor type). The converters may thus determine the same at least one respiratory parameter from different sensor inputs, e.g. such that different units 20 can receive input from different sensor types, and derive the same at least one respiratory parameter therefrom. Therefore, in different settings, e.g. on different imaging and/or therapy modalities and/or in different imaging/treatment rooms, the locally available sensor input (for example taking into account constraints of that particular imaging/treatment modality) can be acquired to determine the same respiratory parameter(s), which can be used to guide the subject toward the reference breathing pattern characterization as defined with respect to that/those parameter(s).

The processor 14 is adapted to, e.g. programmed and/or configured to, in a first session 90 of the multisession series, determine when a predetermined or selected breathing goal has been reached based on the at least one respiratory parameter monitored via the input 11.

For example, the device may comprise a goal selector 15 to, automatically or by interaction with an operator, e.g. via the interface 19, select the breathing goal from a plurality of different candidate breathing goals (in the first session 90).

The predetermined or selected breathing goal may define at least one target criterium for the at least one respiratory parameter for use in determining when the breathing goal has been reached, and optionally also in an initial feedback process to guide the patient to or at least toward this goal.

The processor may also be adapted to provide feedback to the subject via the feedback output 12 before the breathing goal has been reached, in which this feedback is based on the at least one respiratory parameter monitored via the input 11. Thus, feedback may be provided to influence the subject's breathing to meet and/or evolve toward the breathing goal.

The processor 14 is adapted to, e.g. programmed and/or configured to, in a first session 90 of the multisession series, determine and store, using the data storage unit 13, a reference breathing pattern characterization of the monitored at least one respiratory parameter once it is determined that the breathing goal has been reached.

The breathing pattern characterization may be qualitatively different from the predetermined or selected goal, e.g. may relate to a different element or subset of the at least one respiratory parameter, may offer a more complete characterization of the breathing than the definition of the goal as such, and/or may be specifically dependent on the observed breathing pattern. For example, a breathing goal may be defined as "shallow breathing," which may be formalized in a criterium such as a breathing amplitude below a predetermined threshold or the minimization of the breathing amplitude, while the breathing pattern characterization may take the form of a timeseries representing the breathing pattern over a (e.g. average) breathing cycle and/or a combination of relevant parameter values, such as breathing cycle duration or rate, maximum amplitude, maximum inspiration and exhalation levels, and the like.

The processor 14 is further adapted to, in at least one subsequent session 91 of the multisession series, retrieve the stored reference breathing pattern characterization from the data storage unit 13, and provide feedback to the subject via the feedback output 12 to influence the subject's breathing while monitoring the respiration via the input 11 in order to match and/or evolve toward the reference breathing pattern characterization.

Likewise, the processor may also be adapted to provide such feedback to the subject via the feedback output 12 based on the at least one respiratory parameter monitored via the input 11 to influence the subject's breathing to match and/or evolve toward the reference breathing pattern characterization in the first session, i.e. after it is determined that the breathing goal is reached and the reference breathing pattern characterization has been determined.

Thus, the device may, in operation, provide sensory feedback to the subject being imaged and/or treated about a determined reference breathing pattern characterization, e.g. one or more preferred values of the at least one breathing parameter for matching the stored reference pattern, and/or its current breathing behavior, e.g. present (or e.g. averaged over a short time) values of the at least one breathing parameter. The preferred value(s) may typically be different from the current value(s) of the breathing parameter(s), but can evolve toward or approximate the preferred value(s) by following the provided guidance.

The feedback mechanism(s) that are implemented by the device may, for example, use a reward function that is based on the monitored values of the breathing parameter(s), e.g. such that the reward is higher in response to the breathing parameter(s) moving closer to the earlier determined reference and lower in response to the parameter moving further from the reference. The feedback mechanism(s) may also take other factors into account, e.g. to balance the reward for matching the reference with a detected ability of the subject to breathe according to the reference breathing pattern. Other parameters associated with this feedback, e.g. a speed or other value representing an ability to evolve toward the reference pattern, may also be stored (and optionally updated in different sessions), e.g. using the storage unit 13, for reuse in later sessions.

The device may also comprise a trigger signal output 16, and the processor 14 may be adapted to generate a trigger signal for indicating that the breathing goal has been reached (e.g. in the first session) and/or that the reference breathing pattern characterization has been matched by the subject's breathing (e.g. in the subsequent session). This trigger signal may be provided to an imaging or therapeutical system via the trigger signal output 16, such as to commence with a scanning or therapy operation upon receipt of the trigger signal.

The processor 14 may also be adapted to generate a pause signal via the trigger signal output 16 (not necessarily using a same signal line and/or signaling mechanism though) to interrupt the scanning or therapy operation when the subject's breathing deviates from the reference breathing pattern characterization.

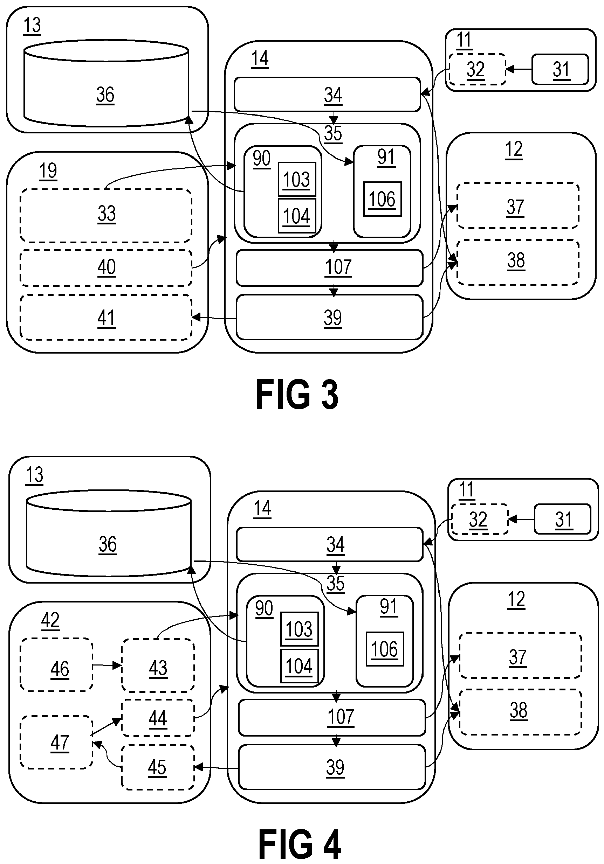

Embodiments of the aspects of the present invention are illustrated by the example shown in Fig. 3. An input 11 receives data 31, e.g. respiratory sensor data, and optionally converts 32 (if not already in a suitable form) the data into the at least one respiratory parameter, for example to accommodate potentially different sensors or sensor types as input.

A breathing coach processor 14 receives the at least one respiratory parameter, e.g. as a live data stream, and processes this information 34.

The processor 14 further determines 35 a goal or target for providing breathing guidance. For a first session of a series, a goal behavior may be selected 33 by an operator via the user interface 19, e.g. by selecting a purpose of the breathing guidance throughout the multisession series.

For a first session 90, upon having the goal defined, the processor may initiate an initial feedback step to guide the patient toward this goal.

Once it is detected 103 that the breathing goal has been reached, the processor determines 104 a reference breathing pattern characterization 36 from the at least one respiratory parameter (optionally by aggregating over a short time, e.g. by averaging over a predetermined time window), and stores the reference breathing pattern characterization 36 in a data storage unit 13, e.g. a patient information database. This data storage unit may be external, e.g. on a central server accessed via a data communication network, such that different processors 14 can access this data in different sessions of the series.

For a further session 91 of the series, the processor determines 35 the target for providing breathing guidance by retrieving 106 the stored reference breathing pattern characterization 36 from the data storage unit 13, e.g. where it may be indexed by a patient and/or multisession series identifier.

The processor 14 further provides 107 feedback to the subject via a feedback output 12 to influence the subject's breathing while monitoring the respiration via the input 11 in order to match and/or evolve toward the reference breathing pattern characterization, e.g. coaches the subject on or towards the desired target breathing pattern characterization 36. This action is at least performed in the subsequent session(s) 91, but may also be executed in the first session 90, e.g. after the reference has been established. As already mentioned, feedback may also be provided during the initial phase of the first session, e.g. before the reference is established, but in this case toward the more generic (e.g. selected 33) goal behavior until the reference can be determined. For example, the feedback output 12 may comprise, or may be operably connected to, a patient-facing display, actuator, audio system and/or other sensory output device. For example, the desired target reference breathing behavior may be presented 37 to the subject, e.g. visualized, displayed, provided via audio cues, and/or in another suitable form. The subject may also (additionally or alternatively) be provided 38 with (bio)feedback on his/her current breathing behavior and/or on the relation between that current behavior and the desired reference breathing pattern. For example, the processor 14 may analyze 39 the correctness of the breathing in relation to the reference breathing pattern characterization to determine suitable feedback 38 for the subject, e.g. by comparing the current with the desired breathing.

The operator may furthermore interact with the operation via the user interface, e.g. to start, stop and/or interrupt 40 the breathing guidance process(es), and or by monitoring 41 the breathing guidance and/or checking the performance of the subject with respect to the reference breathing pattern.

A further example is illustrated in Fig. 4, which is generally similar to the example in Fig. 3. However, in this example, the method/device/computer-program product in accordance with embodiments of the present invention may interact directly with an imaging and/or therapy system 42, e.g. such as to allow efficient automation. Obviously, this does not necessarily exclude a combination with a user interface 19 as shown in Fig. 3.

This automation interface with the imaging/therapy system 42 may provide various optional integrations. For example, the initial goal of the breathing guidance for the first session 90 may be determined automatically by receiving information from the scanner or therapy system. In other words, the imaging/therapy system may determine 43 the breathing goal (and communicate this information to the breathing coach system) or information may be received from the system from which the processor determines a suitable breathing goal. For example, a selected scanner protocol or selected scanner parameters may reveal the purpose of the examination, and thus determine a suitable goal for the breathing guidance. For example, the configured scan protocol and/or initial imaging data 46 may be taken into account to determine a suitable breathing goal for the first session.

The scanner/therapy system 42 may provide a signal to start, stop and/or interrupt 44 the breathing guidance process(es), and/or may receive 45 (a) trigger signal(s) (or a more qualified signal, e.g. a compliance variable representing compliance with the reference breathing on a non-binary or continuous scale) from the breathing guidance process(es) to start, stop and/or interrupt the imaging and/or therapy operation, for example based on the analysis 39 performed by the processor 14 of the correctness of the breathing in relation to the reference breathing pattern characterization. For example, a current imaging and/or therapy system state 47 may be used to start and/or stop the breathing guidance, and/or the analysis 39 of the correctness of the current breathing with respect to the reference, determined by the breathing guidance processes, may be used to affect (e.g. change) the current imaging and/or therapy system state 47, e.g. by interrupting the operation of a scanner or therapy system when performance of the breathing with respect to the reference target is poor.

Interaction with the imaging/therapy system and/or another auxiliary system may also retrieve additional information to be used by the breathing guidance. For example, live imaging data from the imaging/therapy system or other data acquired the system (e.g. a navigator signal) may be used to analyze the present breathing behavior, e.g. in lieu of a respiratory sensor or as additional information to improve the accuracy. The imaging/therapy system may also receive a camera feed to monitor the patient during the procedure, which may also be provided to the breathing guidance implementing system as sensor data or in addition to other sensor data to improve the accuracy.

Furthermore, in a prolonged procedure or multisession series, a treatment (or generic conditions, such as weight gain/loss), may cause gradual changes in breathing, e.g. due to changes of anatomical structures and/or the patient's condition. In embodiments of the present invention, consistent changes in the breathing behavior may be detected, e.g. when the breathing behavior settles, during the feedback phase, at a different level than represented by the reference and/or when a deviation from the reference increases over time during a session. The method (and/or processor of a device) in accordance with embodiments may therefore be adapted to detect such change, and update the stored reference breathing pattern characterization accordingly. Such change may also be flagged, e.g. in the data storage, to indicate that revision of a treatment plan may be required or may need to be considered.

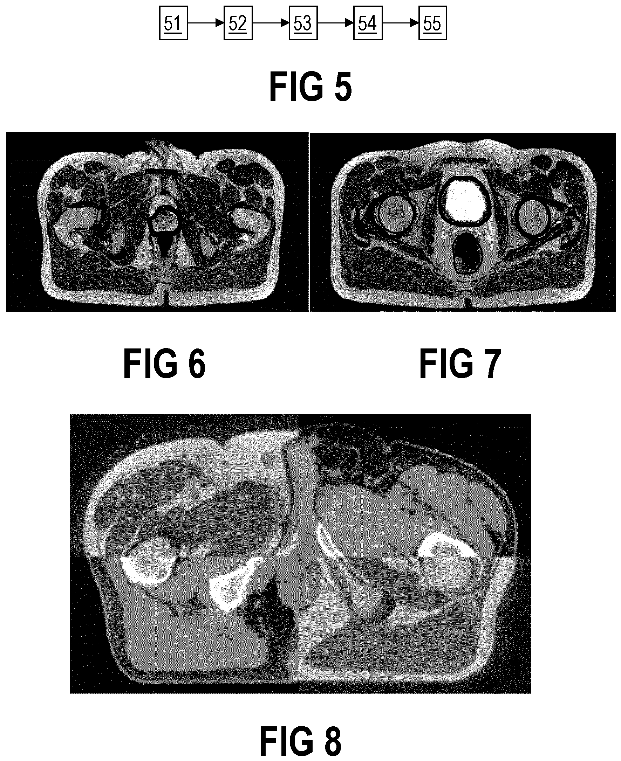

Advantages of embodiments of the present invention can be illustrated by a typical radiotherapy workflow, e.g. as illustrated in Fig. 5. In a first stage, a diagnosis 51 and staging of a cancer condition is determined, and a radiotherapy treatment may be proposed as therapeutic strategy. A dose and fractionation scheme may then be proposed 52 by a radiation oncologist. The treatment target is then typically localized 53 on the basis of at least one, often a plurality of, image sets from one or more imaging modalities, e.g. CT and MRI, for example by defining the contours of the cancerous tissue in the (e.g. co-registered) images, as well as nearby organs at risk (OAR). While CT imaging offers valuable information with respect to ionizing radiation transport through the body, which is obviously important for planning a radiotherapy, MRI images may offer important additional information, e.g. due to a superior soft tissue contrast compared to (conventional) CT images.

This information is used to determine 54 a detailed treatment plan, e.g. defining the dose, delivery angles, delivery times, fractions, etc.. The sequence concludes by performing 55 the actual radiotherapy session(s), e.g. typically (or often) in multiple fractions spread in time. It will be understood that this illustrated workflow is a mere simplified representation of an often more complex reality, e.g. to emphasize the role of MRI and/or CT imaging in the workflow.

In the step of contouring 53 the treatment target(s) and/or organ(s) at risk, CT and MR imaging may thus be used. While CT images can provide the electron density information, used for the radiotherapy dose calculation, MR images may offer a better soft tissue contrast and therefore are often better suited to contour the target and the organs at risk. For example, Fig. 6 shows a (slice of) a contour map of a treatment target, while Fig. 7 shows a contour map of organs at risk.

To calculate a treatment plan in step 54, the contours made on (e.g.) the MR images are copied to the CT images. To project the contours from MR images to CT images, registration between the two image types is typically necessary. Fig. 8 shows a checkerboard mosaic of the MRI (top-left, bottom-right) and CT (top-right, bottom-left) images after being registered. The image registration determines a common coordinate system between two image sets in order to correlate a patient's anatomical and functional information. The two image types may be registered by using landmarks, such as bony structures, using the body contour or by using fiducial markers. Many other registration techniques are known in the art, such as relying on a mutual information measure or the like. The registration process may define a simple affine transformation relating one of the images(ets) to the other, or a more detailed mapping, e.g. using a deformation grid, the latter of which may be more accurate in compensation non-rigid deformations between the state of the subject in both images, but may also be more susceptible to errors, e.g. due to overfitting.

However, the registration step can be computationally quite challenging, and to achieve good results it may rely on the patient position in both imaging modalities to be the same, or at least as nearly as possible. To achieve this, patient position devices (PPDs) can for example be used, such as thermoplastic head masks to fix the head of the patient to the table, vacuum cushions and/or knee rests that are put on a specific, indexed (i.e. reproducible) location.

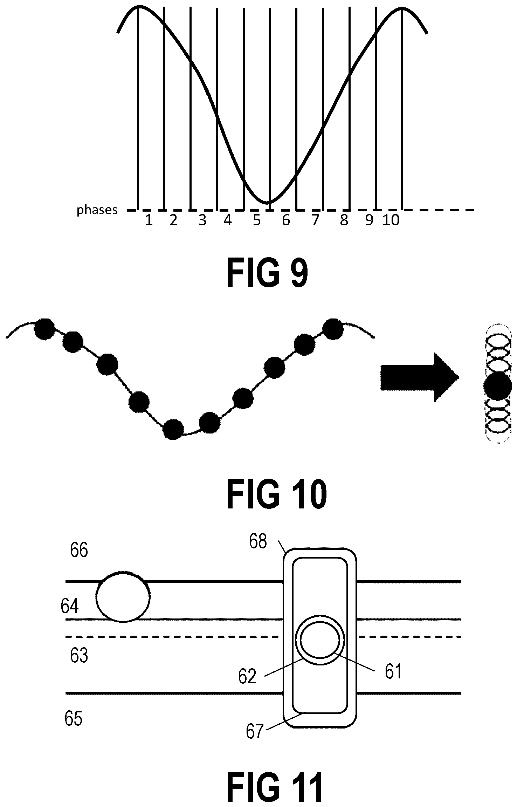

The image data may be acquired at different points in the breathing cycle. Fig. 9 schematically illustrates a breathing cycle, with 10 different time frames indicated as "phases." The data required for reconstructing images can be measured under "free breathing" conditions, e.g. without careful control or influencing of the patient's breathing, in, for example, an image acquisition that can take several minutes. Breathing measurements on CT may typically be performed by a breathing belt apparatus, or another type of breath sensing, such as camera-based respiration monitoring. Due to the breathing movement, a blurring will occur in the image if a single image is reconstructed from the acquired data, or (preferably) the sensor data may be collated with the acquired image data such as to identify the corresponding breathing cycle phase, such that for each phase a specific image can be reconstructed.

To image targets in the body that move due to breathing motion, e.g. of the thorax and abdomen area, the patient may be instructed to hold breath while acquiring the image data, but more 4D CT imaging is used. Breath holds (BHs) may for example be used in cases in which an organ at risk (e.g. the heart) is positioned in the radiation beam and therefore could be exposed to a radiation dose that is too high. By using a BH (in inspiration) the organ will move into a more "safe" zone which will decrease the dose in the healthy tissue. This approach may for example be applied in breast treatments and the pediatric treatment of Hodgkin disease.

As already mentioned, four-dimensional (4D) CT (and/or MRI) imaging may be used in which different 3D images are reconstructed corresponding to different breath phases, e.g. a cine of the respiratory cycle can be reconstructed. The breathing cycle of the patient can thus be divided into 10 phases (or another number of phases), as illustrated in Fig. 9. For 4D MRI, a similar approach can be followed, using again measurements of the breathing cycle by suitable devices, such as breathing belts or camera (and appropriate camera video processing). In magnetic resonance imaging, alternatively a radiofrequency (RF) navigator signal can be used to measure the respiratory cycle, which is also referred to as "auto-navigation." In this approach, the phase information from the scanning data is used to monitor the respiratory cycle and bin the data accordingly for 4D reconstruction.

It thus becomes clear that quality of a 4D imaging series heavily depends on the way a patient is breathing during the image data acquisition from which the 4D series is to be reconstructed. Regular breathing, e.g. at a consistent frequency and amplitude, may lead to 4D image data with better image quality. Not only is a reproducible (or accurately reproduced) patient positioning important while acquiring data by different imaging modalities, e.g. both on CT and MRI, to enable a high quality registration between the two image types (e.g. between 4D image sets of different modality), but the breathing behavior of the patient also affects the quality, i.e. should also be preferably accurately reproduced to facilitate a good registration between the 4D image sets. Therefore, a need exists in the art for providing good means and methods to ensure a reproducible breathing pattern across imaging modalities.

For planning the actual treatment, e.g. for calculating a dose map, an "average" image of the volume targeted may be created (also referred to as "untag" image), as schematically illustrated in Fig. 10. This (time or breathing cycle) averaged image can be used to contour the internal target volume (ITV) consisting of an internal margin added to the clinical target volume (CTV), e.g. to compensate for internal physiologic movement and variations in size, shape, and position of the CTV. Referring to Fig. 11, the CTV defines a margin around the gross target volume (discernable tumor) to indicate the suspected extent of inflected tissue around the visible tumor. Over each breathing cycle, the target (CTV/ITV) will oscillate around a geometric average position 63, which is not necessarily equal to the time-weighted average position 64 (taking e.g. a non-constant speed and/or an asymmetric breathing cycle into account). This causes the position of the CTV to be smeared out (in time) between its position at maximum inhale 65 and maximum exhale 66, which is taken into account by defining the internal target volume 67. The treatment modality (e.g. technical constraints) may add another margin onto the ITV, defining the planning target volume PTV 68.

In step 55 of the schematic workflow, the patient is treated. Currently, breathing behavior is most often not taken into account during the treatment. However, by coaching the breathing behavior during treatment towards the same pattern as during imaging, in accordance with embodiments of the present invention, the dose delivery may be improved. For example, the "untag" image will form a basis for a better (e.g. more accurate) a priori representation of the dose delivery distribution to be expected in practice, since the averaging (or otherwise aggregating) of the breath phase components to form the untag image will better represent the time-averaged physical location of the target. Likewise, planning can potentially be optimized, e.g. in particular by reducing the margins on the ITV (and thus also PTV). Excessive radiation exposure of healthy tissue to harmful radiation can therefore also be potentially reduced.

Other features, or details of the features described hereinabove of a device (resp. computer-program product) in accordance with embodiments of the present invention shall be clear in view of the description provided hereinabove relating to a method in accordance with embodiments of the present invention.

## Claims

1. A device (10) for providing breathing guidance to a subject during multiple diagnostic imaging and/or therapy sessions of a multisession series for said subject, the device comprising:
- an input (11) for receiving sensor data and determining at least one respiratory parameter therefrom to continually monitor the respiration of the subject during each of said sessions,
- a feedback output (12) for providing sensory feedback to the subject in each of said sessions,
- a data storage unit (13), and
- a processor (14),
wherein said processor (14) is adapted to, in a first session (90) of said multisession series,
- determine when a predetermined or selected breathing goal has been reached based on the at least one respiratory parameter monitored via the input (11), and
- determine and store, using the data storage unit (13), a reference breathing pattern characterization of the monitored at least one respiratory parameter once it is determined that the breathing goal has been reached,
wherein said processor (14) is further adapted to, in at least one subsequent session (91) of said multisession series,
- retrieve said stored reference breathing pattern characterization from the data storage unit (13), and
- provide feedback to the subject via the feedback output (12) to influence the subject's breathing while monitoring the respiration via the input (11) in order to match and/or evolve toward the reference breathing pattern characterization.

2. The device of claim 1, wherein said processor is adapted to, in said first session (90), before it is determined that said breathing goal is reached, provide feedback to the subject via the feedback output (12) based on the at least one respiratory parameter monitored via the input (11) in order to influence the subject's breathing to meet and/or evolve toward said breathing goal.

3. The device of claim 1 or claim 2, wherein said processor is adapted to, in said first session (90), after it is determined that said breathing goal is reached, provide feedback to the subject via the feedback output (12) based on the at least one respiratory parameter monitored via the input (11) in order to influence the subject's breathing to match and/or evolve toward the reference breathing pattern characterization.

4. The device of any of the previous claims, comprising a goal selector (15) to, in said first session (90), automatically or by interaction with an operator select said breathing goal from a plurality of different candidate breathing goals, wherein said breathing goal defines at least one target criterium for said at least one respiratory parameter for use in determining when said breathing goal has been reached.

5. The device of any of the previous claims, comprising a trigger signal output (16), wherein said processor (14) is adapted to generate a trigger signal for indicating that said breathing goal has been reached and/or said reference breathing pattern characterization has been matched by the subject's breathing to an imaging or therapeutical system via said trigger signal output (16), such as to commence with a scanning or therapy operation upon receipt of said trigger signal

6. The device of claim 5, wherein said processor (14) is adapted to generate a pause signal via the trigger signal output (16) to interrupt the scanning or therapy operation when the subject's breathing deviates from the reference breathing pattern characterization.

7. The device of any of the previous claims, wherein said device comprises multiple separate units (20), each unit comprising an instance of said input (11), said feedback output (12) and said processor (14), the units being operably connected to said data storage unit (13), such that different units have access to the reference breathing pattern characterization and can respectively operate in different rooms and/or in combination with different imaging and/or therapy systems for provide breathing guidance in different sessions of the multisession series.

8. The device of claim 7, wherein at least two of said inputs (11) comprise a converter (17) adapted to receive sensor input from respectively different sensors and for determining the same at least one respiratory parameter therefrom, such that different units can receive input from different sensor types and derive the same at least one respiratory parameter therefrom.

9. A method (100) for providing breathing guidance to a subject during multiple diagnostic imaging and/or therapy sessions of a multisession series for said subject, the method comprising:
- during each of said sessions, monitoring (102) the respiration of the subject by repeatedly measuring at least one respiratory parameter,
- in a first session (90) of said multisession series, determining (103) when a predetermined or selected breathing goal has been reached based on the monitored at least one respiratory parameter,
- in said first session, determining (104) and storing a reference breathing pattern characterization of the monitored at least one respiratory parameter once it is determined (103) that the breathing goal has been reached,
- in at least one subsequent session (91) of said multisession series, retrieving (106) the stored reference breathing pattern characterization, and
- in said at least one subsequent session, providing (107) feedback to the subject to influence the subject's breathing while monitoring (102) the respiration in order to match or evolve toward the reference breathing pattern characterization.

10. The method of claim 9, comprising, in said first session (90), providing (105) feedback to the subject before it is determined that said breathing goal is reached in order to influence the subject's breathing while monitoring (102) the respiration to meet or evolve toward said breathing goal.

11. The method of any of claims 9 to 10, wherein said step of providing (107) feedback to the subject to influence the subject's breathing to match or evolve toward the reference breathing pattern characterization is also performed in the first session after determining (104) the reference breathing pattern characterization.

12. The method of any of the claims 9 to 11, comprising, in said first session (90), automatically or by interaction with an operator, selecting (101) said breathing goal from a plurality of different candidate breathing goals, wherein said breathing goal defines at least one target criterium for said at least one respiratory parameter.

13. The method of any of the claims 9 to 12, comprising generating (109) a trigger signal for indicating that said breathing goal has been reached (103) to an imaging or therapeutical system, such as to commence with a scanning or therapy operation upon receipt of said trigger signal.

14. The method of any of the claims 9 to 13, comprising, in said at least one subsequent session (91), determining (108) when the earlier stored reference breathing pattern is matched or sufficiently approximated by the subject's breathing and then generating (110) a further trigger signal for an imaging or therapeutical system so as to commence with a scanning or therapy operation upon receipt of said further trigger signal.

15. A computer-program product for performing, when executed on a computer, the method of any of the previous claims.
